# EUROPEAN PATENT APPLICATION

(11) **EP 1 477 190 A1**
(43) Date of publication of application: **17.11.2004**
(21) Application number: 03380114.3
(22) Date of filing: 12.05.2003
(51) Int. Cl.: A61L 27/04

(54) **Porous block of metallic spheres for growing bone tissue on the inside**

(71) Applicant: Lafitt, S.A., 46988 Paterna (Valencia) (ES)
(72) Inventor: Forriol Pico, Joaquin LAFITT, S.A., II Fase 46988 Paterna (Valencia) (ES); Barbera Tomas, David LAFITT, S.A., II Fase 46988 Paterna (Valencia) (ES); Mas Gomez, Francesc LAFITT, S.A., II Fase 46988 Paterna (Valencia) (ES); Moreno Ballester, José Francisco LAFITT, S.A., II Fase 46988 Paterna (Valencia) (ES)
(74) Representative: Canadell-Isern, Roberto

(57) **Abstract**

Porous block for growing bone tissue on the inside; this block is made of a metallic material or alloy, and it is singular in that it is porous. This porosity is characterized by the following: (a) it is continuous throughout the block; (b) it is formed by the residual space resulting from joining small spheres of a metallic material or alloy, the size of which ranges from 300 to 600 µm and which are connected to each other by means of a neck, providing the material with a porosity ranging from 30 to 60% (Fig. 11).

## Description

### OBJECT OF THE INVENTION

The object of the invention protected in this Patent is a "Porous block for growing bone tissue on the inside".

This block is made of a metallic material or alloy, and it is singular in that it is porous. This porosity is characterized by the following: (a) it is continuous throughout the block; (b) it is formed by the residual space resulting from joining small spheres of a metallic material or alloy, the size of which ranges from 300 to 600 µm and which are connected to each other by means of a neck. These spheres provide the material with a porosity ranging from 30 to 60%.

### BACKGROUND OF THE INVENTION

As reference to the current state-of-the-art, it should be noted that, up until now, numerous surgical implants have used porous coatings of different characteristics to facilitate or induce the growth of bone tissue on the surface of the implant, thus improving attachment of the implant to the adjacent osseous structures.

However, it seems obvious that if a porous surface coating facilitates bone tissue growth and attachment on the coated surface, an implant that has continuous porosity throughout will notably improve the levels of bone growth and attachment, thus optimising the function of the implant.

### DESCRIPTION OF THE INVENTION

The "Porous block for growing bone tissue on the inside" is formed by joining small spheres of a metallic material, the size of which ranges from 300 to 600 µm and which are connected to each other by means of a neck. These spheres provide the material with a porosity ranging from 30 to 60%.

In this way, the osseous tissue will penetrate and pass through the block and will become attached to its surface, thus achieving greater resistance upon extraction of the implant.

A preferential way to execute this type of block is in the form of an intersomatic box. The intersomatic fusion consists of a bone fusion of two adjacent vertebral bodies through the space interposed between them, known as the discoid space because it is occupied under normal conditions by the inter-vertebral disk. After extirpating the disk, a bone graft, interposed between the vertebral bodies, has been used to occupy the disk space and to favour fusion. But the isolated bone is structurally unable to withstand the transmitted load and the graft often collapses. To prevent this, the use of prefabricated boxes made of metal or another firmer material has become common practice in recent years. These boxes are filled with bone and thus succeed in greatly increasing the resistance of this type of graft.

However, these boxes are very unstable during the period immediately after the operation, precisely when there is a greater need for stability to favour bone growth. However, by using a box made on the basis of a porous block such as the one described above, both bone growth and attachment of the box to the adjacent vertebrae will be greater, thus favouring intersomatic fusion.

On the other hand, if the pore permits bone growth through the box, this obviates the need for inserting a graft into the box. Therefore, one of the variants of this invention would be a compact porous box that does not include a prefabricated housing for the graft.

To complete the description of this invention and facilitate interpretation of its formal, structural and functional characteristics, the attached drawings schematically show different aspects of a preferential execution of the "Porous block for growing bone tissue on the inside", which is the object of this patent.

### DESCRIPTION OF THE FIGURES

The figures are as follows:

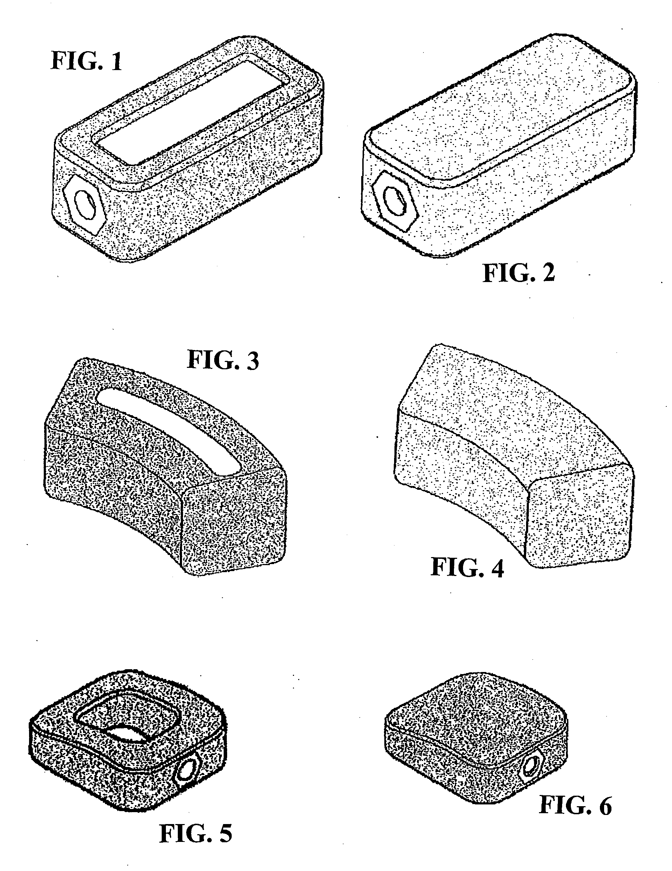

- Figure 1 shows a view in perspective of a posterior lumbar intersomatic box made of porous block.
- Figure 2 shows a view in perspective of a posterior lumbar intersomatic box made of porous block, without a prefabricated housing for the graft.
- Figure 3 shows a view in perspective of a posterior trans-foraminal intersomatic box made of porous block.
- Figure 4 shows a view in perspective of a posterior trans-foraminal intersomatic box made of porous block, without a prefabricated housing for the graft.
- Figure 5 shows a view in perspective of a fore lumbar intersomatic box made of porous block.
- Figure 6 shows a view in perspective of a fore lumbar intersomatic box made of porous block, without a prefabricated housing for the graft.

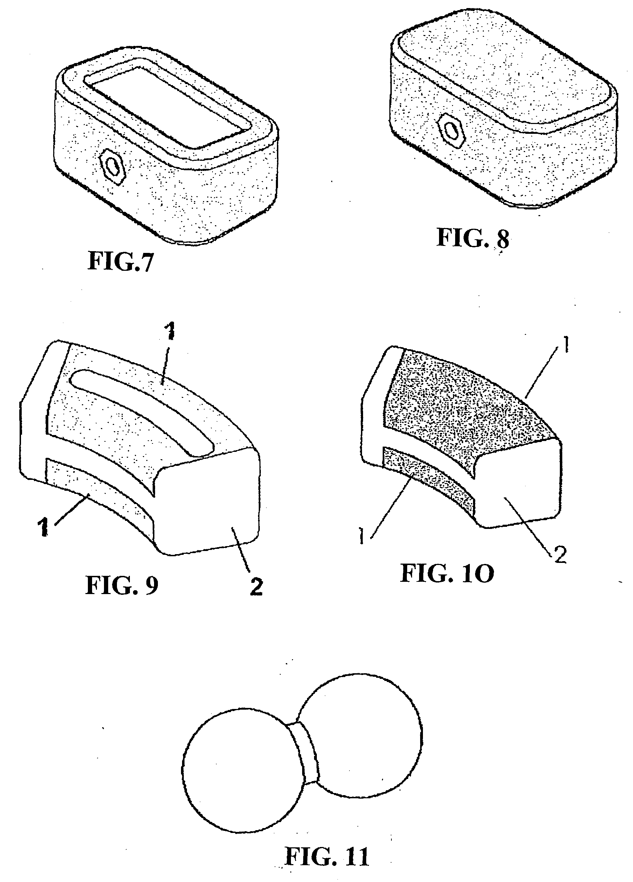

- Figure 7 shows a view in perspective of a fore cervical intersomatic box made of porous block.
- Figure 8 shows a view in perspective of a fore cervical intersomatic box made of porous block, without a prefabricated housing for the graft.
- Figure 9 shows a view in perspective of a posterior trans-foraminal intersomatic box composed of two porous blocks (1) attached to a non-porous metal structure (2) (e.g., a titanium alloy), so that it is the structure that absorbs a certain type of forces, such as implant impaction forces.
- Figure 10 shows a view in perspective of a posterior trans-foraminal intersomatic box composed of two porous blocks (1) attached to a non-porous metal structure (2) (e.g., a titanium alloy), so that it is the structure that absorbs a certain type of forces, such as implant impaction forces, without a prefabricated housing for the graft.
- Figure 11 shows how two of the spheres that form the implant material are joined by means of a metallurgic neck.

## Claims

1. POROUS BLOCK FOR GROWING BONE TISSUE ON THE INSIDE, essentially **characterized by** the fact that this porous block is formed by joining small spheres of a metallic material, the size of which ranges from 300 to 600 µm, and by connecting these spheres to each other by means of a neck, providing the material with a porosity that ranges from 30 to 60%.

2. POROUS BLOCK FOR GROWING BONE TISSUE ON THE INSIDE, as per Claim 1, **characterized by** the fact that it consists of a body whose component spheres are of the same size.

3. POROUS BLOCK FOR GROWING BONE TISSUE ON THE INSIDE, as per Claim 1, **characterized by** the fact that it consists of a body whose component spheres are not of the same size.

4. POROUS BLOCK FOR GROWING BONE TISSUE ON THE INSIDE, as per Claim 1, **characterized by** the fact that it consists of a body that contains an osteo-conductive or osteo-inductive biomaterial in its pores.

5. POROUS BLOCK FOR GROWING BONE TISSUE ON THE INSIDE, as per Claim 1, **characterized by** the fact that it consists of a parallelepiped suitable for use as an implant for the spinal column, more specifically as a posterior lumbar intersomatic box, as a posterior trans-foraminal intersomatic box, as a fore lumbar intersomatic box, or as a fore cervical intersomatic box.

6. POROUS BLOCK FOR GROWING BONE TISSUE ON THE INSIDE, as per Claims 1 and 5, **characterized by** the fact that this parallelepiped does not contain a prefabricated housing for the graft.

7. POROUS BLOCK FOR GROWING BONE TISSUE ON THE INSIDE, as per Claim 1, **characterized by** the fact that it consists of a body attached to a non-porous metal structure, for example a titanium alloy, so that it is the structure that absorbs a certain type of forces, such as implant impaction forces.

8. POROUS BLOCK FOR GROWING BONE TISSUE ON THE INSIDE, as per Claims 1 and 7, **characterized by** the fact that this body does not contain a prefabricated housing for the graft.
